# EUROPÄISCHE PATENTSCHRIFT

(11) **EP 1 713 749 B1**
(45) Veröffentlichungstag und Bekanntmachung des Hinweises auf die Patenterteilung: **23.07.2008**
(21) Anmeldenummer: 04805021.5
(22) Anmeldetag: 23.12.2004
(51) Int. Cl.: C07C 6/02, C07C 45/50, C07C 29/16, C07C 11/02

(54) **VERFAHREN ZUR HERSTELLUNG VON OLEFINEN MIT 8 BIS 12 KOHLENSTOFFATOMEN**
METHOD FOR THE PRODUCTION OF OLEFINS COMPRISING 8 TO 12 CARBON ATOMS
PROCEDE POUR PRODUIRE DES OLEFINES AVEC 8 A 12 ATOMES DE CARBONE

(30) Priorität: 14.02.2004 DE 102004007289; 08.07.2004 DE 102004033410
(43) Veröffentlichungstag der Anmeldung: 25.10.2006
(73) Patentinhaber: Evonik Oxeno GmbH, 45772 Marl (DE)
(72) Erfinder: WIESE, Klaus-Diether, 45721 Haltern am See (DE); KAIZIK, Alfred, 45772 Marl (DE); MASCHMEYER, Dietrich, 45657 Recklinghausen (DE); BÜSCHKEN, Wilfried, 45721 Haltern am See (DE); SCHÜLLER, Ulf, 44139 Dortmund (DE)
(74) Vertreter: Hirsch, Hans-Ludwig
(86) Internationale Anmeldenummer: PCT/EP2004/053693
(87) Internationale Veröffentlichungsnummer: WO 2005/080302

(56) Entgegenhaltungen:
- EP-A- 0 488 073
- WO-A-91/09826
- WO-A-92/13818
- DE-A1- 3 232 557
- DE-A1- 10 041 345
- DE-A1- 10 241 266
- GB-A- 1 330 112
- GB-A- 2 070 638
- US-A- 5 723 712

## Beschreibung

Die vorliegende Erfindung betrifft die Herstellung von Olefinen oder Olefingemischen mit 8 bis 12 Kohlenstoffatomen durch eine vierstufige Synthese aus einem oder mehreren Olefin(en) mit 4 bis 6 Kohlenstoffatomen.

Olefine mit 8 bis 12 Kohlenstoffatomen sind begehrte Einsatzstoffe in der chemischen Industrie. Aus ihnen können beispielsweise durch Hydroformylierung um ein Kohlenstoffatom längere Aldehyde gewonnen, die ihrerseits wieder für wichtige technische Produkte Einsatz finden. Beispiele für die Verwendung von Aldehyden ist die Hydrierung der Aldehyde zu den Alkoholen und deren Umsetzung mit Carbonsäuren zu Estern. So führt beispielsweise die Veresterung von C₉-Alkoholen mit Phthalsäureanhydrid zu Diisononylphthalaten, die sehr begehrte Weichmacher in der kunststoffverarbeitenden Industrie sind. Ein technisch wichtiges Verfahren, welches angewendet wird, ist außerdem die Oxidation der Aldehyde zu den entsprechenden Carbonsäuren, die unter anderem zu öllöslichen Metallsalzen umgesetzt werden können. Diese werden beispielsweise als Trocknungsbeschleuniger für Lacke (Sikkative) oder Stabilisatoren für PVC eingesetzt. Weiterhin dienen Alkohole, insbesondere C₁₃-Alkohole, als Vorstufe für Tenside.

Eine weitere beispielhafte technische Anwendung ist die Umsetzung von Olefinen unter Katalyse von starken Säuren mit Kohlenmonoxid und Wasser zu den um ein Kohlenstoffatom längeren Carbonsäuren, die unter dem Namen KOCH-Reaktion Eingang in die Literatur gefunden hat. Dabei werden tertiäre verzweigte Carbonsäuregemische erhalten, die wegen ihrer verzweigten Natur wiederum sehr gut zur Herstellung der zuvor erwähnten Metallsalze geeignet sind. Ein besonders wichtige Verwendung der tertiären Carbonsäuren ist die Umsetzung mit Acetylen zu Vinylestern, die als Comonomere zur inneren Weichmachung von Polymeren dienen. Copolymere von Vinylestern tertiärer Carbonsäuren mit Vinylacetat sind beispielsweise die Basis für wasserdispergierbare umweltfreundliche Farben und Lacke und energiesparende Wärmeschutzputze von Gebäuden.

Von den oben genannten Verwendungen der C₈- bis C₁₂-Olefine ist deren Nutzung zur Herstellung von Weichmacheralkoholen die bedeutendste.

Olefine mit 8 bis 12 Kohlenstoffatomen können durch Oligomerisierung von Ethylen, Propylen oder Gemischen davon gewonnen werden. Dabei entsteht als primäres Produkt ein Gemisch von Olefinen mit unterschiedlicher C-Zahl. Aus diesen Gemischen können die gewünschten Olefine abgetrennt werden. Die unerwünschten Olefine mit einer niedrigeren oder höheren Anzahl an Kohlenstoffatomen, können nur durch mehrstufige Synthesen in die Zielolefine umgewandelt werden. Nachteilig für die Herstellung von Olefinen mit 8 bis 12 Kohlenstoffatomen auf diesem Weg ist der hohe Preis für Ethylen und/oder Propylen.

Olefine mit 8 bis 12 Kohlenstoffatomen können auch durch Dimerisierung von Olefinen oder Olefingemischen mit 4 bis 6 Kohlenstoffatomen gewonnen werden. Insbesondere hat die Dimerisierung von C₄-Olefinen zu Gemischen isomerer Octene große technische Bedeutung.

Für die technische Dimerisierung von Olefinen mit 4 bis 6 Kohlenstoffatomen gibt es im Wesentlichen drei Verfahren, die sich durch die verwendeten Katalysatorsysteme unterscheiden. Lange bekannt ist die Oligomerisierung an sauren Katalysatoren (Verfahren A), wobei technisch z. B. Zeolithe oder Phosphorsäure auf Träger eingesetzt werden. Hierbei werden im wesentlichen Isomerengemische von mehrfach verzweigten Olefinen erhalten (WO 92/13818). Ein ebenfalls weltweit ausgeübtes Verfahren ist die Oligomerisierung mit löslichen Ni-Komplexen, bekannt als DIMERSOL-Verfahren (Verfahren B) (B. Cornils, W.A. Herrmann, Applied Homogenous Catalysis with Organometallic Compounds, Seite 261 - 263,Verlag Chemie 1996). Schließlich ist noch die Oligomerisierung an Nickel-Festbett-Katalysatoren zu erwähnen, wie z. B. das Verfahren der OXENO Olefinchemie GmbH. Das Verfahren hat Eingang in die Literatur als OCTOL-Prozess (Verfahren C) (Hydrocarbon Process., Int. Ed. (1986) 65 (2. Sect.1), Seiten 31 - 33; J. Schulze, C4-Hydrocarbons and Derivates, Springer Verlag, Berlin 1989, Seiten 69 - 70) gefunden.

Selbst wenn man von einem reinen Olefin oder einem Olefingemisch, bei dem sich die einzelnen Olefine nur durch die Lage der Doppelbindung und deren Konfiguration unterscheiden, ausgeht, erhält man bei der Dimerisierung keinen reinen Stoff, sondern ein Gemisch von vielen Strukturisomeren, die sich ihrerseits wieder aus praktisch allen Doppelbindungsisomeren in unterschiedlichen Anteilen zusammensetzen, wobei viele Doppelbindungsisomeren noch zusätzlich eine cis/trans-Isomerie zeigen. Diese Konstitutions- und Konfigurationsisomere können je nach Herstellungsverfahren in unterschiedlichen Anteilen vorliegen.

Wird beispielsweise Dibuten ausgehend von Raffinat II (C₄-KW-Fraktion ohne Butadien und Isobuten) oder Raffinat III (Raffinat II nach vollständiger oder teilweiser Abtrennung von 1-Buten) hergestellt, erhält man Olefingemische von im Wesentlichen unverzweigten, einfach verzweigten und zweifach verzweigten Grundstrukturen. Die folgenden Daten dienen nur der Orientierung, da je nach Verfahrensbedingungen wechselnde Anteile der einzelnen Strukturgruppen erhalten werden.

Ein Maß für den Verzweigungsgrad ist der Isoindex. Er ist definiert durch die Anzahl der Verzweigungen pro Molekül. Demnach haben lineare Octene (n-Octene) einen Isoindex von 0, Methylheptene einen von 1 und Dimethylhexene einen von 2. Bei der Berechnung des Isoindex von Gemischen sind die Massenanteile der einzelnen Verbindungsgruppen zu berücksichtigen.

**Tabelle 1: Zusammensetzung der Dimerisierungsgemische (Dibutene) ausgehend von Raffinat III in Abhängigkeit vom ausgeübten Verfahren.**

| | Verfahren A | Verfahren B | Verfahren C |
|---|---|---|---|
| | Zeolithkatalyse | Dimersol | Octol |
| n-Octene | ∼ 0% | ∼ 6 % | ~13% |
| 3-Methyl-Heptene | ∼ 5% | ~59% | ~62% |
| 3,4-Dimethylhexene | ~70% | ~34% | ~24% |
| Sonstige C₈-Olefine | ~25% | ~1% | ~ 1% |
| Isoindex | > 1,9 | ≅ 1,29 | ≅ 1,12 |

Werden an Stelle von Raffinat II oder Raffinat III andere noch Isobuten enthaltende C₄-Schnitte wie das Raffinat I eingesetzt, werden darüber hinaus eine Fülle weiterer, noch verzweigterer Strukturen gebildet, im wesentlichen Trimethyl-Pentene wie 2,2,4-Trimethyl-Pentene, 2,2,3-Trimethyl-Pentene, 2,3,4 Trimethyl-Pentene, 2,3,3-Trimethyl-Pentene und andere mehr erhalten. Solche Dibutene mit einem Isoindex von größer 2 sind auch unter dem Namen "Codibutylen" bekannt.

Die anwendungstechnischen Eigenschaften der beispielsweise aus Dibuten hergestellten Folgeprodukte sind oftmals von der Zusammensetzung und insbesondere vom Verzweigungsgrad des eingesetzten Olefins abhängig. Dies kann ganz extreme Formen annehmen, wie an folgenden Beispielen zu erkennen ist.

Ein wichtiges Anwendungsgebiet von Dibutenen ist die Herstellung von C₉-Alkoholen, die ihrerseits mit Carbonsäuren verestert werden. So erhält man aus Dibuten Isononanolgemische und durch deren Veresterung mit Phthalsäureanhydrid Isononylphthalate, welches als Weichmacher in Kunststoffen eingesetzt werden.

Der Verzweigungsgrad der Isononylketten der Phthalate steht in einem engen Zusammenhang mit dem Verzweigungsgrad des eingesetzten Olefins, so dass die Eigenschaften der Phthalate wesentlich durch die Struktur des eingesetzten Olefingemisches mitbestimmt sind

**Tabelle 2: Vergleich typischer dynamischer Viskositäten technisch eingesetzter Nonylphthalate, mit Co-HD: Klassisches Kobalt-Hochdruckverfahren, 200 - 300 bar, 140 - 180 °C Rh-HD:Rhodium-Hochdruckverfahren, 150 - 300 bar, 120 - 130 °C, unmodifizierter oder mit Triphenylphosphinoxid modifizierter Rhodiumkatalysator**

| Rohstoff | Oligomerisierungsverfahren | Hydroformylierungsverfahren | Viskositäten der Isononylphthalate (20 °C) |
|---|---|---|---|
| Raffinat I | A | Co-HD | ≅ 165 mPa s |
| Raffinat II oder III | A | Co-HD | 116-120 mPa s |
| Raffinat II oder III | B oder C | Co-HD | 70-85 mPa s |
| Raffinat II oder III | B oder C | Rh-HD | 90-100 mPa s |

Im Markt werden als Weichmacher bevorzugt (Iso-)Nonylphthalate mit niedriger Viskosität, insbesondere mit einer Viskosität von unter 85 mPa*s, verlangt. Nonylphthalate mit einer solchen Viskosität können nur aus Nonanolen hergestellt werden, die aus C₄-Schnitten, die weniger als 5 Massen-% Isobuten bezogen auf die C₄-Olefinfraktion enthalten, und üblicherweise durch Dimerisierung nach Verfahren B oder C gewonnen werden.

In technischen C₄ bis C₆-Kohlenwasserstoffgemischen, insbesondere in C₄- oder C₅-Schnitten aus Olefincrackern, liegen lineare und verzweigte Olefine nebeneinander vor. Die Abtrennung der verzweigten Olefine ist aufwändig und meistens nur nach Derivatisierung möglich. So wird beispielsweise Isobuten nach Umsetzung mit Methanol als MTBE aus einem C₄-Schnitt abgetrennt.

Die Verwendung von tertiären Ethern, insbesondere MTBE, als Octanzahlverbesserer in Ottokraftstoffen wird wegen einer möglichen Grundwasserkontamination zunehmend kritisch gesehen, beispielsweise wird der Zusatz von MTBE in Ottokraftstoffen demnächst in Kalifornien verboten. Es ist nicht auszuschließen, dass auch in anderen Staaten die Verwendung von tertiären Ethern eingeschränkt werden könnte.

Um die Rohstoffbasis für C₈- bis C₁₂-Olefine, die für die Herstellung von Weichmacher- oder Tensidalkoholen besonders gut geeignet sind, zu vergrößern und die Menge an Kopplungsprodukte, deren zukünftige Vermarktung unsicher ist, zu verringern, bestand die Aufgabe der vorliegenden Erfindung darin, ein Verfahren zu entwickeln, das in der Lage ist auch die in den technischen Gemischen vorliegenden verzweigten C₄- bis C₆-Olefine zu nutzen und/oder das eine Bildung von hochsiedenden Nebenprodukten, also Verbindungen mit mehr als 12 Kohlenstoffatomen, weitgehend vermeidet und/oder das einfach durchzuführen ist

Überraschenderweise wurde nun gefunden, dass die Umsetzung von einem oder mehreren linearen und/oder verzweigten Olefin(en) zu einem oder mehreren Olefine(n) mit C-Zahlen, die der Summe der C-Zahlen von zwei gleichen oder unterschiedlichen im Einsatzgemisch vorliegenden Olefine entspricht, durch eine einfache mehrstufige Synthese erreicht werden kann, die folgende Reaktionsschritte umfasst:
a) Hydroformylierung des Ausgangsolefins oder der Ausgangsolefine,
b) Hydrierung des/der im Reaktionsschritt a) entstandenen Aldehyds/Aldehyde zu dem/den entsprechenden Alkohol(en),
c) Wasserabspaltung aus dem/den im Verfahrensschritt b) entstandenen Alkohol(en) zu dem/den dem/den entsprechenden 1-Olefin(en) und
d) Metathese der/des im Schritt (c) entstandenen 1-Olefins/1-Olefine unter Ethylenabspaltung zu einem oder mehreren Olefin(en) mit C-Zahlen, die der um zwei verminderten Summe der Anzahl an Kohlenstoffatomen von zwei gleichen oder unterschiedlichen miteinander reagierenden Olefinen entspricht.

Gegenstand der vorliegenden Erfindung ist demnach ein Verfahren zur Herstellung von einem oder mehreren Olefin(en) mit 8 bis 12 Kohlenstoffatomen aus einem oder mehreren Olefin(en) mit 4 bis 6 Kohlenstoffatomen durch eine vierstufige Synthese,
dadurch gekennzeichnet, dass
a) im ersten Reaktionsschritt das Einsatzolefin oder Einsatzolefingemisch hydroformyliert wird,
b) der/die im ersten Reaktionsschritt a) erhaltenen Aldehyd(e) zu dem/den entsprechenden Alkohol(en) hydriert wird/werden,
c) aus dem/den im zweiten Reaktionsschritt erhaltenen Alkohol(en) durch Wasserabspaltung ein oder mehrere 1-Olefin(e) hergestellt wird/werden und
d) aus dem/den im dritten Reaktionsschritt anfallenden 1-Olefin(en) durch Metathese unter Ethylenabspaltung ein oder mehrere Olefin(e) gewonnen wird/werden.

DE 32 32 557 A1 betrifft ein Verfahren zur Herstellung eines Alkohols durch Hydroformylierung, wobei diese in zwei Stufen durchgeführt wird.

EP 0 488 073 A betrifft ein Verfahren zur Herstellung von Isoocten durch Dimerisation von n-Buten.

US 5,723,712 A beschreibt die Dimerisation von Olefinen an einem speziellen Katalysatorsystem.

GB 2 070 638 A offenbart ein Verfahren zur Herstellung von Isoocten durch Dimerisation eines C4-Stroms, wobei enthaltene Butadiene selektiv hydriert werden.

In WO 91/09826 A wird die Herstellung eines Gemisches von isomeren Octenen durch Dimerisation von n-Buten an einem Nickeloxid-Katalysator beschrieben.

In GB 1 330 112 A wird die Herstellung von Isononanol durch Hydroformylierung von 2-Ethylhexen-1 beschrieben. Das Produkt wird in Form der Phthalat- oder Adipat-Ester als Weichmacher in PVC-Kunststoffen eingesetzt.

DE 100 41 345 A1 betrifft ein Verfahren zur Herstellung langkettiger α-Olefine durch isomerisierende Metathesereaktion einer linearen C₄-C₁₀ Fraktion.

Der Vorteil des erfindungsgemäßen Verfahren liegt darin, dass sowohl lineare als auch verzweigte Olefine mit 4 bis 6 Kohlenstoffatomen oder ihre Gemische zur Herstellung von Olefinen mit 8 bis 12 Kohlenstoffatomen eingesetzt werden können. Die gemäß dem erfindungsgemäßen Verfahren hergestellten Zielolefine können endständig hydroformyliert werden und ergeben nach Hydrierung der Aldehyde primäre Alkohole mit 9 bis 13 C-Atomen, die sich insbesondere für die Herstellung von Weichmachern und/oder Tensiden eignen. Da im gegensatz zu üblichen Verfahren im erfindungsgemäßen Verfahren auch verzweigte Olefine oder Gemische mit verzweigten Olefinen zur Herstellung von C₈-C₁₂-Olefinen mit innenständigen Doppelbindungen eingesetzt werden können, verbreitert sich die Rohstoffbasis zur kostengünstigen Herstellung entsprechender Alkohole mit 9 bis 13 Kohlenstoffatomen. Diese können als Weichmacheralkohole z- B. zur Herstellung von Phthalaten, die sich durch niedrige Viskosität auszeichnen, verwendet werden.

Durch das erfindungsgemäße Verfahren ist es also nicht mehr notwendig verzweigte Olefine, wie z. B. Isobuten aus dem Olefingemisch, z. B. als MTBE abzutrennen. Auf diese Weise wird die Abhängigkeit der Wirtschaftlichkeit des Verfahrens von einem speziellen Koppelprodukt vermieden. Im Fall der Herstellung von C₈-Olefinen aus C₄-Olefinen kann, wie später gezeigt wird, die Herstellung eines Koppelproduktes vollständig vermieden werden.

Ein weiterer Vorteil des erfindungsgemäßen Verfahrens zur Herstellung von Olefinen mit 8 bis 12 Kohlenstoffatomen aus Olefinen mit 4 bis 6 Kohlenstoffatomen besteht darin, dass im Gegensatz zu einem Verfahren, bei dem ein Oligomerisierungsschritt (Di- oder Trimerisierungsschritt) von Olefinen vorhanden ist, der zur Bildung von Nebenprodukten mit höheren Kohlenstoffatom-Zahlen, wie z. B. Tri- oder Oligomeren führt, solche unerwünschten Nebenprodukte nicht anfallen.

Mit dem erfindungsgemäßen Verfahren ist es bei der Herstellung von C₈-Olefinen, die zu Weichmacheralkoholen aufgearbeitet werden, außerdem möglich je nach vorliegenden Rohstoffen 1-Buten, Isobuten oder 2-Butene jeweils allein oder als Mischung die eine oder mehrere dieser Verbindungen aufweist, als Einsatzstoff zu verwenden. Das erfindungsgemäße Verfahren vergrößert also die Rohstoffbasis sowie die Flexibilität.

Die Erfindung macht sich die an sich bekannten Verfahren der Hydroformylierung, Hydrierung, Wasserabspaltung und Metathese zu nutze. Durch die erfindungsgemäße Verkettung der einzelnen Reaktionsschritt zu einem Verfahren zur Herstellung von Olefinen mit 8 bis 12 Kohlenstoffatomen aus Olefinen mit 4 bis 6 Kohlenstoffatomen werden die genannten Vorteile erzielt.
Der erfindungsgemäße Verfahren und die mit ihm hergestellten Produkte werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Zum besseren Verständnis des erfindungsgemäßen Verfahrens werden die Verfahrensschritte a) bis d) zusätzlich an Hand einer erfindungsgemäßen Behandlung eines C₄-Olefine aufweisenden Kohlenwasserstoffstroms erläutert, ohne dass die Erfindung darauf beschränkt sein soll.

Das erfindungsgemäße Verfahren zur Herstellung zumindest eines Olefins mit 8 bis 12 Kohlenstoffatomen aus zumindest einem Olefin mit 4 bis 6 Kohlenstoffatomen durch eine vierstufige Synthese, zeichnet sich dadurch aus, dass
a) in einem im ersten Verfahrensschritt das zumindest eine Einsatzolefin hydroformyliert wird,
b) der im ersten Schritt a) erhaltenen zumindest eine Aldehyd in einem zweiten Verfahrensschritt zu dem entsprechenden Alkohol hydriert wird,
c) aus dem im zweiten Verfahrensschritt b) erhaltenen zumindest einem Alkohol durch Wasserabspaltung zumindest ein 1-Olefin hergestellt wird und
d) aus dem im dritten Verfahrensschritt c) erhaltenen zumindest einem 1-Olefin(en) durch Metathese unter Ethylenabspaltung zumindest ein Olefin gewonnen wird.

Das erfindungsgemäße Verfahren kann so durchgeführt werden, dass nur ein Olefin eingesetzt oder aber eine Mischung von Olefinen eingesetzt wird. Insbesondere kann ein Gemisch von Olefinen mit 4 bis 6 Kohlenstoffatomen eingesetzt werden und entsprechend ein Gemisch von Olefinen mit 8 bis 12 Kohlenstoffatomen erhalten wird. Wird im Rahmen der vorliegenden Erfindung von "einem Olefin" gesprochen, so wird darunter nicht nur ein einzelnes Molekül sondern eine Gruppe, wie z. B. ein Strom, von olefinisch ungesättigten Verbindungen verstanden, die die gleiche Bruttoformel aufweisen und optional ein und dieselbe Struktur aufweisen. Wird im Rahmen der vorliegenden Erfindung von Olefinen gesprochen, so sind darunter Gruppen/Ströme von olefinisch ungesättigten Verbindungen zu verstehen, die eine unterschiedliche Anzahl von C-Atomen und/oder eine unterschiedliche Struktur aufweisen.

Im erfindungsgemäßen Verfahren werden insbesondere Kohlenwasserstoffgemische eingesetzt, die Monoolefine mit 4 bis 6 Kohlenstoffatomen aufweisen, jedoch vorzugsweise praktisch frei sind von mehrfach ungesättigten Kohlenwasserstoffen, wie z. B. Dienen oder Acetylenderivaten. Es können im erfindungsgemäßen Verfahren Kohlenwasserstoffgemische eingesetzt werden, die Olefine mit drei unterschiedlichen Kohlenstoffatom-Zahlen, die Olefine mit zwei unterschiedlichen Kohlenstoffatom-Zahlen oder Olefine mit gleicher Kohlenstoffatom-Zahl aufweisen.

Die Olefine selbst bzw. die Olefine aufweisenden Kohlenwasserstoffgemische, die im erfindungsgemäßen Verfahren eingesetzt werden können, können aus den unterschiedlichsten Quellen stammen. Olefine mit 6 C-Atomen sind beispielsweise in Leichtsiederfraktionen, die bei der Aufarbeitung von Erdöl anfallen, enthalten. Eine Quelle für Olefine mit 5 Kohlenstoffatomen ist der C₅-Schnitt aus Olefincrackern.

Im erfindungsgemäßen Verfahren werden bevorzugt Kohlenwasserstoffe mit 4 Kohlenstoffatomen eingesetzt. Insbesondere können technische C₄-Schnitte eingesetzt werden, die einen Isobuten-Gehalt von größer 3 Gew.%, vorzugsweise größer 10 Gew.-% und besonders bevorzugt größer 20 Gew.% aufweisen. Die bedeutendste Quelle für C₄-Olefine ist der C₄-Schnitt des Crackbenzins aus Steamcrackern. Daraus wird nach Extraktion(sdestillation) des Butadiens oder dessen Selektivhydrierung zu einem n-Butengemisch ein Kohlenwasserstoffgemisch (Raffinat I oder hydriertes Crack-C₄) hergestellt, das Isobuten, 1-Buten und die beiden 2-Butene enthält. Ein anderer Rohstoff für C₄-Olefine ist der C₄-Schnitt aus FCC-Anlagen, der wie oben beschrieben aufgearbeitet werden kann. C₄-Olefine, hergestellt durch Fischer-Tropsch-Synthese, sind nach Selektivhydrierung des darin enthaltenden Butadiens zu n-Butenen ebenfalls ein geeigneter Einsatzstoff. Darüber hinaus können Olefingemische, die durch Dehydrierung von C₄-Kohlenwasserstoffen oder durch Metathesereaktionen erhalten werden, oder andere technische Olefinströme geeignete Einsatzstoffe sein.

Wegen des häufig sehr hohen Trennaufwands werden die im als Einsatzgemisch einzusetzenden technischen Gemisch vorliegenden Olefine im Allgemeinen nicht getrennt, sondern die Gemische werden direkt in der Verfahrensstufe a) eingesetzt.

### Verfahrensstufe a)

Die Hydroformylierung aller Olefine im Reaktionsgemisch kann in einer Stufe erfolgen. Dazu wird ein Katalysator benötigt, der vermag, Olefine mit unterschiedlicher Lage der Doppelbindung und/oder Anzahl der Verzweigungen zu hydroformylieren. Katalysatoren, die dafür geeignet sind, bewirken aber meistens nur eine geringe Selektivität für die Bildung von Produkten (Aldehyde, Alkohole, Formiate), die durch endständige Hydroformylierung entstanden sind, und/oder zeigen eine zu geringe Reaktionsgeschwindigkeit für ein technisches Verfahren.

Wenn die Endprodukte (Olefine mit 8 bis 12 Kohlenstoffatomen) hauptsächlich als Vorstufe für Weichmacheralkohole und Tensidalkohole genutzt werden sollen, ist es zweckmäßig, die Hydroformylierung derart durchzuführen, dass ein hoher Anteil an Produkten, die durch endständige Hydroformylierung entstanden sind, gewonnen wird; denn nur die endständig hydroformylierten Produkte weisen den gleichen Verzweigungsgrad wie ihre Ausgangsolefine auf, bei mittelständiger Hydroformylierung dagegen erhöht sich der Verzweigungsgrad des entstandenen Produkts um 1.

Die in einem technischen Gemisch vorliegenden Olefine unterscheiden sich in ihrer Reaktivität bei der Hydroformylierung beträchtlich Im Allgemeinen sind Olefine mit endständigen Doppelbindungen reaktiver als Olefine mit mittelständigen Doppelbindungen und lineare Olefine reaktiver als verzweigte. Speziell im Falle der C₄-Olefine gilt, 1-Buten ist reaktiver als Isobuten und Isobuten ist reaktiver als die beiden 2-Butene. Diese unterschiedliche Reaktivität kann genutzt werden, um einen hohen Anteil an Produkten, die durch endständige Hydroformylierung entstanden sind, zu gewinnen, d. h., aus 1-Buten soll hauptsächlich Valeraldehyd und nicht 2-Methylbutanal, aus Isobuten 3-Methylbutanal und nicht 2,2-Dimethylpropanal sowie aus den beiden 2-Butenen möglichst viel Valeraldehyd (Pentanal) und wenig 2-Methylbutanal entstehen. Da es bislang keinen Katalysator gibt, der simultan mit zufriedenstellender Geschwindigkeit sowohl die Umsetzung von 1-Buten als auch von Isobuten und den 2-Butenen zu Produkten, die durch endständige Hydroformylierung entstanden sind, bewirkt, wird die Hydroformylierung vorzugsweise zumindest zweistufig durchgeführt.

In einer ersten Stufe wird die Hydroformylierung mit einem geeigneten Katalysator unter Bedingungen vorgenommen, bei denen nur α-Olefine (1-Buten, Isobuten), jedoch nicht die 2-Butene zu den entsprechenden Aldehyden umgesetzt werden. Dabei werden die Bedingungen derart gewählt, dass 1-Buten möglichst selektiv zu Valeraldehyd und Isobuten möglichst selektiv zu 3-Methylbutanal umgesetzt wird Als Katalysatoren werden beispielsweise Verbindungen eingesetzt, die Rhodium und triorganische Phosphorverbindungen, insbesondere Phosphine als Liganden aufweisen. Die Umsetzung kann in homogener Phase (analog dem UCC-Verfahren EP 0 562 451) oder in heterogener Phase (analog dem Rhone-Poulenc-Ruhrchemie-Verfahren DE 026 27 354, EP 0 562 451) durchgeführt werden. Wegen der leichteren Katalysatorabtrennung wird diese erste Stufe des Verfahrensschrittes a) bevorzugt gemäß dem zweiten Verfahren durchgeführt. Die Reaktionstemperaturen betragen bevorzugt von 70 bis 150 °C, vorzugsweise von 100 bis 130 °C. Die Verfahrensdrücke betragen vorzugsweise von 2 bis 20 MPa, bevorzugt von 3 bis 6 MPa.

Optional kann die Hydroformylierung der 1-Olefine im Mehrphasensystem, wobei Edukt, Produkt und Synthesegas in einer kontinuierlichen Katalysatorphase dispergiert sind, unter hohen Leerrohrgeschwindigkeiten durchgeführt werden. Solche Verfahren werden beispielsweise in DE 199 25 384 A1 und DE 199 57528 A1 beschrieben, auf welche hier ausdrücklich verwiesen wird.

Die Hydroformylierung der 1-Olefine kann einstufig oder zweistufig durchgeführt werden. Bei der zweistufigen Hydroformylierung wird im ersten Reaktor vorwiegend 1-Buten und im zweiten Reaktor hauptsächlich Isobuten umgesetzt. In beiden Reaktoren können die gleichen Katalysatoren oder unterschiedliche eingesetzt werden. Bei Verwendung gleicher Katalysatoren ist eine gemeinsame Katalysatoraufarbeitung möglich.

Nach der gerade beschriebenen Hydroformylierung von 1-Buten und Teilen des Isobutens in der ersten Stufe des Verfahrensschrittes a) verbleiben im Einsatz-Kohlenwasserstoffgemisch die 2-Butene und gegebenenfalls Isobuten und höchstens Spuren von 1-Buten. Dieses Gemisch kann als solches unter Verwendung eines anderen Katalysatorsystems oder nach Auftrennung in zwei Fraktionen, von denen eine Isobuten und die andere die beiden 2-Butene enthält, hydroformyliert werden. Bevorzugt wird das Gemisch aufgetrennt und die Isobuten aufweisende Fraktion und die 2-Butene aufweisende Fraktion werden getrennt hydroformyliert.

Optional wird die Fraktion, die als Olefine hauptsächlich 2-Butene enthält, zu überwiegend C₈-Olefinen oligomerisiert. Dies erfolgt mit Hilfe von Nickel-haltigen-Katalysatoren, vorzugsweise Festbettkatalysatoren, wie beispielsweise im OCTOL-Prozess (siehe oben).

Das Isobuten bzw. die Isobuten aufweisende Fraktion kann in hohen Selektivitäten zu 3-Methylbutanal hydroformyliert werden. Geeignete Katalysatoren dafür sind Rhodiumkomplexe, die ein- oder mehrzähnige Phosphitliganden enthalten. Geeignete einzähnige Phosphitliganden sind beispielsweise Triarylphosphite, deren Arylgruppen sowohl in ortho-Stellung zum Phosphit-Sauerstoff eine sperrige Gruppe aufweisen als auch in m- oder p-Stellung substituiert sind, wie z. B. Tris(2,4-di-tert.-butyl-phenyl)phosphit Die Hydroformylierung von Isobuten unter Verwendung eines Katalysatorsystems, das aus Rhodium und einem Bisphosphit besteht, wird beispielsweise in den Patentschriften US 4,668,651, US 4,769,498 und WO 85/03702 beschrieben, auf welche ausdrücklich verwiesen wird und deren Offenbarungsgehalt Gegenstand der vorliegenden Beschreibung ist.

Optional kann die abgetrennte Isobutenfraktion teilweise in die vorgeschalteten Hydroformylierungsstufen zurückgeführt werden. Werden vom Isobuten die gesättigten Kohlenwasserstoffe getrennt, kann es vollständig zurückgeführt werden.

Die Hydroformylierung von 2-Butenen bzw. 2-Butenen aufweisenden Fraktionen kann mit Hilfe von verschiedenen Katalysatoren durchgeführt werden, wobei üblicherweise ein Gemisch aus 2-Methylbutanal und Valeraldehyd entsteht. In den meistens Fällen ist 2-Methylbutanal das Hauptprodukt. Die Verwendung von unmodifizierten Kobaltkatalysatoren als Katalysator für die Hydroformylierung von 2-Butenen wird in EP 0 646 563 und die Verwendung von unmodifiziertem Rhodium wird in EP 0 562 451 beschrieben. Weiterhin kann für die Hydroformylierung von 2-Butenen das gleiche Katalysatorsystem, das für die Hydroformylierung von Isobuten eingesetzt wird, nämlich ein Komplex aus Rhodium und einzähniges Triarylphosphit verwendet werden. Hohe Selektivitäten an Valeraldehyd können bei der Verwendung eines Katalysators, bestehend aus Rhodium und sperrigen aromatischen Bisphosphiten, erhalten werden, wie sie beispielsweise in EP 0 213 639 beschrieben werden. Allerdings sind die Reaktionsgeschwindigkeiten für ein technisches Verfahren gering.

Wie oben ausgeführt ist, können die im Einsatzstoff vorliegenden Olefine getrennt oder gemeinsam hydroformyliert werden. Wenn der Linearität der Endprodukte keine große Bedeutung zukommt, ist es zweckmäßig die Olefine gemeinsam zu hydroformylieren. Ist dagegen ein möglichst wenig verzweigtes Endprodukt erwünscht, ist es erforderlich, die Hydroformylierung in mindestens zwei Stufen durchzuführen. Im Falle eines C₄-Olefingemisches bedeutet der letztere Fall, dass im ersten Reaktor 1-Buten und gegebenenfalls Isobuten umgesetzt wird und in dem/den nachgeschalteten Reaktor(en) die restlichen Olefine.

Aus den Hydroformylierungsgemischen wird nach bekannten Verfahren der Katalysator abgetrennt. Beispielsweise kann bei Verfahren, bei denen der Rhodium-Katalysator homogen im Reaktionsgemisch vorliegt, der Katalysator destillativ abgetrennt werden. Bei der Umsetzung in heterogener Phase (zwei flüssige Phasen) erfolgt die Abtrennung des Katalysators durch Phasentrennung (Ed. B. Cornils, W. A. Herrmann, Applied Homogeneous Catalysis with Organic Compounds, Vol. 1, S. 80, VCH-Verlag, 1996).

Die Hydroformylierungsgemische können nach der Entkatalysierung entweder direkt in Verfahrensstufe b) eingesetzt werden oder aber destillativ oder mit anderen Trennmethoden in zwei oder mehrere Fraktionen aufgetrennt werden. Insbesondere kann es vorteilhaft sein, das Hydroformylierungsgemisch so aufzuarbeiten, dass eine oder mehrere im wesentlichen Aldehyde aufweisende Fraktionen erhalten werden.

### Verfahrensschritt b)

Die entkatalysierten Hydroformylierungsgemische oder die daraus durch ein Trennverfahren wie z. B. Destillation abgetrennten Aldehyde oder Aldehyd aufweisenden Fraktionen werden im zweiten Verfahrensschritt b) des erfindungsgemäßen Verfahrens hydriert. Dabei können die Hydroformylierungsgemische getrennt oder gemeinsam hydriert werden. Durch die Hydrierung entstehen aus den Aldehyden die entsprechenden gesättigten Alkohole. Dies sind beispielsweise n-Pentanol, 2-Methylbutanol und 3-Methylbutanol.

Zur Hydrierung können z. B. Nickel-, Kupfer-, Kupfer/Nickel-, Kupfer/Chrom-, Kupfer/Chrom/Nickel-, Zink/Chrom-, Nickel/Molybdän-Katalysatoren verwenden werden. Die Katalysatoren können trägerfrei sein, oder die hydrieraktiven Stoffe bzw. ihre Vorläufer können auf Trägern, wie beispielsweise Siliziumdioxid oder Aluminiumoxid, aufgebracht sein. Bevorzugte Katalysatoren, an denen die Hydroformylierungsgemische hydriert werden, weisen jeweils 0,3 bis 15 Massen-% Kupfer und Nickel sowie als Aktivatoren 0,05 bis 3,5 Massen-% Chrom und vorteilhaft 0,01 bis 1,6 Massen-%, vorzugsweise 0,02 bis 1,2 Massen- % einer Alkalikomponente auf einem Trägermaterial, vorzugsweise Aluminiumoxid und Siliziumdioxid auf. Die Mengenangaben beziehen sich auf den noch nicht reduzierten Katalysator. Die Alkalikomponente ist optional. Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudaten oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird im Allgemeinen bei einem Gesamtdruck von 0,5 bis 50 MPa, insbesondere von 1,5 bis 10 MPa durchgeführt. Eine Hydrierung in der Gasphase kann auch bei niedrigeren Drücken durchgeführt werden, wobei dann entsprechend große Gasvolumina vorliegen. Werden mehrere Hydrierungsreaktoren eingesetzt, können die Gesamtdrücke in den einzelnen Reaktoren innerhalb der genannten Druckgrenzen gleich oder verschieden sein. Die Reaktionstemperaturen können bei der Hydrierung in flüssiger oder gasförmiger Phase in der Regel von 120 bis 220 °C, insbesondere von 140 bis 180 °C betragen. Solche Hydrierungen sind beispielsweise in den Patentanmeldungen DE 198 42 369 und DE 198 42 370 beschrieben, auf welche hier ausdrücklich verwiesen wird.

Im erfindungsgemäßen Verfahren wird die Hydrierung bevorzugt in Gegenwart von Wasser durchgeführt. Das benötigte Wasser kann im Reaktorzulauf enthalten sein. Es ist jedoch auch möglich, Wasser an geeigneter Stelle in die Hydrierapparatur einzuspeisen. Bei Gasphasenhydrierung wird Wasser zweckmäßig in Form von Wasserdampf zugeführt. Ein bevorzugtes Hydrierverfahren ist die Flüssigphasenhydrierung unter Zusatz von Wasser, wie sie beispielsweise in DE 100 62 448 beschrieben ist. Besonders bevorzugt wird Hydrierung bei einem Wassergehalt von 0,05 bis 10 Massen-%, insbesondere 0,5 bis 5 Massen-%, ganz besonders 1 bis 2,5 Massen-% durchgeführt. Der Wassergehalt wird dabei im Hydrieraustrag bestimmt.

Die aus der Hydrierung erhaltenen Gemische können entweder direkt in Verfahrensstufe c) eingesetzt werden oder aber destillativ oder mit anderen Trennmethoden in zwei oder mehrere Fraktionen aufgetrennt werden. Insbesondere kann es vorteilhaft sein, das Hydrierungsgemisch so aufzuarbeiten, dass eine oder mehrere im wesentlichen Alkohole mit der gleichen Anzahl an Kohlenstoffatomen aufweisende Fraktionen erhalten werden.

Wenn ausgehend von einem C₄-Kohlenwasserstoffschnitt ein Teil der darin enthaltenen linearen Olefine innenständig hydroformyliert wird, kann es zweckmäßig sein, das daraus durch Hydrierung entstandene 2-Methylbutanol ganz oder teilweise abzutrennen.

### Verfahrensschritt c)

Aus dem nach der Hydrierung gemäß Schritt b) enthaltenen Alkoholgemisch bzw. den Alkoholen oder Alkohol aufweisenden Fraktionen werden in dem dritten Verfahrensschritt c) durch Wasserabspaltung die entsprechenden 1-Olefine hergestellt. Im erfindungsgemäßen Verfahren wird diese Dehydratisierung in der Gas- oder Flüssig/Gas-Mischphase an suspendierten oder stückigen im Festbett angeordneten Katalysatoren kontinuierlich oder diskontinuierlich durchgeführt. Die Wasserabspaltung wird wegen der einfachen Abtrennung der Reaktionsprodukte aus dem Reaktionsgemisch bevorzugt an festen Katalysatoren im Temperaturbereich von 200 bis 500 °C in der Gas- oder Gas/Flüssig-Mischphase durchgeführt. Bevorzugt wird eine kontinuierliche Dehydratisierung an im Festbett angeordneten Katalysator durchgeführt. Als Katalysatoren können Oxide der Erdalkalimetalle, des Aluminiums, Indiums, Galliums, des Siliziums, Scandiums, Yttriums, Lanthans, Titans, Zirkoniums, Thoriums sowie der seltenen Erden verwendet werden. Es können auch Mischoxide und Kombinationen der obigen Oxide eingesetzt werden. Bei einigen Katalysatoren kann durch Zugabe von Alkalioxiden eine bestimmte Acidität eingestellt werden.

Aus der wissenschaftlichen Fachliteratur sind beispielsweise folgende geeigneten Katalysatoren bekannt:
NiO/Al₂O₃; CuO/Al₂O₃; Al₂O₃ (J. Mol. Catal. A. Chem. (1997), 121 (2-3), S. 157-159);
ZrO₂; sulfatisiertes ZrO₂ (J. Mol. Cat. A. Chem (1997), 118 (1), S. 88-89);
Al₂O₃; Co₂O₃; ThO₂; In₂O₃ (J. Catal. (1988), 110 (2), S. 416-418);
HfO₂/ZrO₂ (J. Phys. Chem. (1980), 84 (1), 55-56);
Al₂O₃/Na₂O; ThO2 (J. Catal. (1981), 68 (2), S. 383-387);
ThO₂ (J. Org. Chem. (1967), 32 (11), 3386-3389);
La₂O₃ (Z. Phys. Chem.(1985), 144, S. 157-163);
Ga₂O₃ (J. Org. Chem. (1977), , 44 (13), S. 2142-2145);
ThO₂; Al₂O₃ (J. Org. Chem. (1972), 37 (8), S. 1240-1244);

Vorzugsweise erfolgt die Auswahl der Katalysatoren und der Reaktionsbedingungen so, dass die Bildung von Nebenprodukten, wie beispielsweise von Ethern sowie die Isomerisierung der gebildeten 1-Olefine zu Olefinen mit innenständigen Doppelbindungen weitgehend vermieden wird. Für die Herstellung der 1-Olefine aus den primären Alkoholen durch Wasserabspaltung im Verfahrensschritt c) werden im erfindungsgemäßen Verfahren deshalb bevorzugt basische oder stark basische Katalysatoren eingesetzt Die eingesetzten Katalysatoren können als Hauptkomponenten Aluminiumoxid (Al₂O₃) und/oder Zirkoniumoxid (ZrO₂) sowie Alkalimetall- und/oder Erdalkalioxide aufweisen. Als weitere Komponenten können im Katalysator Titandioxid, Siliziumdioxid und/oder Thoriumoxid mit 0,01 bis 3 Massen-%, bevorzugt 0,5 bis 5 Massen-% enthalten sein.

Der Anteil an basischen Metalloxiden (Hydroxide werden in Oxide umgerechnet) im Katalysator beträgt bevorzugt von 0,01 bis 10 Massen-%, besonders bevorzugt von 0,1 bis 5 Massen-%, insbesondere bevorzugt von 0,1 bis 3 Massen-%. Bevorzugte Alkalimetalloxide sind Natrium- und/oder Kaliumoxid. Als Erdalkalimetalloxide werden bevorzugt Magnesium-, Strontium- und/oder Bariumoxid eingesetzt. Als Katalysator in Schritt c) wird ganz besonders bevorzugt ein mit Bariumoxid (BaO) modifiziertes γ-Aluminiumoxid, welches formal aus Bariumoxid und Aluminiumoxid besteht, verwendet.

Bevorzugt werden γ-Aluminiumoxide mit einer BET-Oberfläche von 80 bis 350 m²/g, bevorzugt 120 bis 250 m²/g (bestimmt durch N₂-Absorption gemäß DIN 66131) eingesetzt. Die Katalysatoren werden nach bekannten Methoden hergestellt. Gängige Methoden sind beispielsweise Fällung, Tränkung oder Besprühung eines Al₂O₃-Körpers mit einer entsprechenden Salzlösung und anschließende Calcinierung.

Ebenso vorteilhaft kann es sein, wenn Katalysatoren eingesetzt werden, wie sie in DE 103 59 628 beschrieben werden und die einen Anteil an Zirkoniumdioxid von 80 bis 99 Massenteile, an Yttriumoxid von 0,5 bis 10 Massenteile und an Erdalkali- oder Alkalioxiden von 0,1 bis 3 Massenteilen aufweisen.

Die Katalysatoren werden vorzugsweise in Form von Kugeln, Tabletten, Zylindern, Strangextrudaten oder Ringen eingesetzt.

Bei der kontinuierlichen Wasserabspaltung können unterschiedliche Verfahrensvarianten eingesetzt werden. Der Verfahrensschritt c) kann z. B. adiabatisch, polytrop oder praktisch isotherm, d. h. mit einer Tempera.turdifl'erenz von typischerweise kleiner als 10 °C, durchgeführt werden. Der Verfahrensschritt kann ein- oder mehrstufig durchgeführt werden. Im letzteren Fall können alle Reaktoren, zweckmäßig Rohrreaktoren, adiabatisch oder praktisch isotherm betrieben werden. Ebenfalls ist es möglich, einen oder mehrere Reaktoren adiabatisch und die anderen praktisch isotherm zu betreiben. Bevorzugt wird die Wasserabspaltung im geraden Durchgang betrieben. Sie kann jedoch auch unter Produktrückführung betrieben werden. Bei Betrieb im geraden Durchgang beträgt die spezifische Katalysatorbelastung 0,01 bis 30 bevorzugt von 0,1 bis 10 kg Alkohol je kg Katalysator und je Stunde. Bei der Wasserabspaltung gemäß Verfahrensschritt c) beträgt die Temperatur in der Katalysatorschicht vorzugsweise von 200 bis 450 °C, insbesondere von 250 bis 350 °C. Die Wasserabspaltung (Dehydratisierung) kann unter vermindertem Druck, Überdruck oder bei Normaldruck durchgeführt werden.

Das Edukt der Verfahrensstufe c) kann in reiner Form oder in Verdünnung in den Dehydratisierungsreaktor gefahren werden. Als Verdünnungsmittel können inerte Gase oder Gasgemische, wie beispielsweise Stickstoff, Wasserstoff, Kohlenmonoxid, Kohlendioxid, Synthesegas, Methan oder Wasserdampf, oder unter Reaktionsbedingungen inerte organische Lösungsmittel, die vom Reaktionsaustrag leicht abgetrennt werden können, eingesetzt werden.

Um eine möglichst hohe Selektivität hin zur 1-Olefin Bildung zu erzielen, hat es sich als vorteilhaft erwiesen, wenn nur ein Teilumsatz des eingesetzten Alkohols angestrebt wird. Bevorzugt wird die Verfahrensstufe c) so ausgeführt, dass der Umsatz im geraden Durchgang 30 bis 90 % beträgt.

Als Produkt der Verfahrensstufe c) wird ein zumindest ein 1-Olefin aufweisendes Gemisch erhalten. Das Reaktionsgemisch wird vorzugsweise in das/die entstandene 1-Olefin(e), den/die primären Alkohol(e) und gegebenenfalls Nebenprodukte, wie beispielsweise Ether oder Carbonylverbindungen, wie z. B. Aldehyde, aufweisende Fraktionen getrennt. Die Trennung kann z. B. durch Destillation erfolgen. Die nach der Trennung erhaltene Olefinfraktion kann optional zu reinem 1-Olefin aufgearbeitet werden. Die Alkohol aufweisende Fraktion, die den nicht umgesetzten Alkohol aufweist, wird vorzugsweise in die Dehydratisierung zurückgeführt. Das beispielsweise bei der Dehydratisierung von 1-Hydroxyalkan (1-Pentanol) als Nebenprodukt entstandene Aldehyd (n-Pentanal) kann nach Hydrierung zum entsprechenden Alkohol wiederverwendet werden und bedingt somit keinen Stoffverlust. Die Hydrierung kann in einer gesonderten Hydriervorrichtung durchgeführt werden. Es kann jedoch vorteilhaft sein, die als Nebenprodukte erhaltenen Aldehyde in die der Dehydratisierung vorgelagerte Hydrierung (Schritt b) einzuspeisen. Durch diese Abtrennung der Aldehyde aus dem bei der Dehydratisierung erhaltenen Gemisch, anschließende Hydrierung und Rückführung der erhaltenen Alkohole in die Dehydratisierung können bei der erfindungsgemäßen Dehydratisierung Olefine in einer besonders hohen Selektivität erhalten werden.

Die zumindest ein 1-Olefin aufweisende Fraktion kann optional fraktioniert werden. Die einzelnen Fraktionen können getrennt in der nächsten Synthesestufe eingesetzt werden. Weiterhin ist es möglich, eine oder mehrere Fraktion(en) nicht mehr in die nächste Synthesestufe einzusetzen, sondern anderweitig zu verwenden. Es ist auch möglich eine Mischung aus zweien oder mehreren der Fraktionen herzustellen und in der nächsten Verfahrensstufe d) einzusetzen.

Auf diese Weise ist es möglich als Produkt der Verfahrensstufe d) Olefingemische zu erhalten, die unterschiedlichste Isoindices aufweisen.

Ist der Ausgangsstoff des erfindungsgemäßen Verfahrens, der in Verfahrensschritt a) eingesetzt wird, beispielsweise ein Kohlenwasserstoffstrom, insbesondere ein C₄-Kohlenwasserstoffgemisch, das Isobuten und lineare Butene aufweist oder aus diesen besteht, wie z. B. Raffinat I oder selektiv hydriertes Crack-C₄, so wird nach Hydroformylierung, Hydrierung und Wasserabspaltung (Verfahrensstufen a) bis c)) ein C₅-Olefingemisch gewonnen, welches die 1-Olefine 1-Penten, 3-Methylbuten(1) und gegebenenfalls 2-Methylbuten(1) aufweist. 1-Penten hat bei Normaldruck einen Siedepunkt von 30 °C, 2-Methylbuten-1 einen von 31,2 °C und 3-Methylbuten(1) einen von 20,1 °C. Auf Grund der deutlich unterschiedlichen Siedepunkte kann 3-Methylbuten(1) nach dem Verfahrensschritt c) leicht von den beiden anderen Isomeren, die in der erhaltenen, Olefine mit fünf Kohlenstoffatomen aufweisenden 1-Olefinfraktion vorliegen, destillativ abgetrennt werden. 3-Methylbuten-1 ist ein Olefin, für das es einige technische Anwendungen gibt Beispielsweise wird es zur Modifizierung von Polypropylen verwendet.

Die teilweise oder vollständige Abtrennung des 3-Methylbutens-1 verringert den Verzweigungsgrad im zurückbleibenden Isomerengemisch. Somit weist auch das im nächsten Syntheseschritt entstehende Zielprodukt (C₈-Olefine) einen geringeren Verzweigungsgrad (Isoindex) auf. Dies ist insbesondere dann vorteilhaft, wenn das Zielprodukt als Vorstufe für Weichmacheralkohole eingesetzt wird.

Die aus den bisherigen Verfahrenschritten a) bis c) erhältlichen Produkte, die bei Einsatz von 1-Buten, Isobuten und/oder 2-Butenen erhalten werden können, können Tabelle 3 entnommen werden.

**Tabelle 3: Produkte der Verfahrensschritte a) bis c)**

| C4-Komponente | Aldehyd (Produkte der Verfahrensstufe a)) | Alkohol (Produkte der Verfahrensstufe b)) | Olefin (Produkte der Verfahrensstufe c)) |
|---|---|---|---|
| 1-Buten | Pentanal | Pentanol | 1-Penten |
| | + 2-Methylbutanal | + 2-Methylbutanol | + 2-Methylbuten-1 |
| 2-Buten | 2-Methylbutanal | 2-Methylbutanol | 2-Methylbuten-1 |
| | + pentanal | + pentanol | + 1-Penten |
| Isobuten | 3-Methylbutanal | 3-Methylbutanol | 3-Methylbuten-1 |
| | (+ 2,2-Dimethylpropanal) | (+ 2,2-Dimethylpropanol) | |

### Verfahrensschritt d)

Das aus Verfahrensschritt c) erhaltene Produkt kann ganz oder teilweise in die Verfahrensstufe d) überführt werden. Das/die in Verfahrensschritt c) erhaltene(n) Olefin(e) werden bevorzugt unter Bedingungen, unter denen praktisch keine Isomerisierung der Einsatzstoffe und/oder der Produkte durch Verschiebung einer Doppelbindung auftritt, im Verfahrensschritt d) metathetisiert. Dabei entsteht/entstehen unter Ethylenabspaltung ein oder mehrere Olefin(e) mit mittelständiger Doppelbindung. Die Kohlenstoffatom-Zahl der entstandenen Olefine ergibt sich aus der um zwei verminderten Summe der beiden miteinander reagierenden 1-Olefine. Wird ein Gemisch von 1-Olefinen mit der gleichen Kohlenstoff-Zahl n eingesetzt, so haben die Produktolefine die Kohlenstoff-Zahl (2n-2). Werden beispielsweise Gemische eingesetzt, die Olefine mit Kohlenstoff-Zahlen von n und m enthalten, so entstehen Produktolefine mit den Kohlenstoff-Zahlen (2n-2), (2m-2) und (n+m-2). Die maximale Anzahl der im Produktgemisch vorhandenen Olefine ist, ohne Berücksichtigung von Konfigurationsisomere, durch die Summe der verschiedenen Linearkombinationen gegeben. Beispielsweise können aus einem 1-Olefingemisch mit den C₅-Olefinen, 1-Penten und 3-Methylbut-len, folgende C₈-Olefine entstehen: Oct-4-en (cis und trans), 2-Methylhept-3-en (cis und trans), und 2,5-Dimethylhex-3-en (cis und trans).

Um ausschließlich lineare C₈-Olefine zu erhalten ist es notwendig, dass der Ausgangsstrom von allen Olefinen mit Ausnahme von 1-Penten befreit wird Eine solche Abtrennung kann z. B. destillativ erfolgen. Sind ausschließlich C₈-Olefine das Ziel der Verfahrensstufe d) so darf das Einsatzgemisch nur Olefine aufweisen, die 5 Kohlenstoffatome aufweisen und deren Doppelbindung endständig ist. Um zu vermeiden, dass aus den endständigen Olefinen durch Isomerisierung innenständige Olefine entstehen, wird die Metathesereaktion vorzugsweise bei Bedingungen durchgeführt, bei denen eine Isomerisierung weitestgehend vermieden wird. Da das 2-Methylbuten-1 häufig keine Metathesereaktion mit sich selbst eingeht, sondern bei Metathesebedingungen zunächst zu 2-Methylbuten-2 und weiter zu 3-Methylbuten-1 isomerisiert werden können, können bei Einsatz von Kohlenwasserstoffgemischen in Verfahrensschritt d), die ausschließlich 2-Methylbuten-1 aufweisen, durch Kreuz- und Homometathese des sich bildenden 2-Methylbuten-2 mit sich selbst bzw. mit 2-Methylbuten-1 oder 3-Methylbuten-1 eine Vielzahl von verschiedenen Verbindungen, insbesondere 3-Methylbuten-2 und Isobuten entstehen. Um dies zu vermeiden werden vorzugsweise nur solche Ströme in den Verfahrensschritt d) geleitet, die ein Verhältnis von 1-Penten zu 2-Methylbuten-1 von größer oder gleich 1 : 1, vorzugsweise von 1,1 : 1 bis 100000 : 1, besonders bevorzugt von 1000 : 1 bis 10000 : 1. Sollte der aus Verfahrensschritt c) erhaltene Produktstrom diese Bedingungen nicht erfüllen kann das Verhältnis von 1-Penten zu 2-Methylbuten-1 entweder durch Zugabe von 1-Penten oder durch Abtrennung zumindest eines Teils des 2-Methylbuten-1, z. B. durch Destillation eingestellt werden.

Die Metathese kann in flüssiger homogener Phase oder heterogen an einem festen Katalysator kontinuierlich oder diskontinuierlich durchgerührt werden. Im erfindungsgemäßen Verfahren erfolgt die Metathese bevorzugt an einem im Festbett angeordneten stückigen Katalysator. Die Umsetzung kann an den bekannten Trägerkatalysatoren, die z. B. Oxide des Wolframs, Molybdäns oder Rhenium enthalten, durchgeführt werden. Um die Isomerisierung der Olefine und die damit verbundene Bildung von Nebenprodukten weitgehend zu vermeiden, werden im erfindungsgemäßen Verfahren bevorzugt Rheniumkatalysatoren eingesetzt.

Als Katalysatoren können beispielsweise die aus der Literatur bekannten Rhenium-Trägerkatalysatoren, Re₂O₇ auf γ-Al₂O₃ oder auf Mischträgern, wie z. B. SiO₂/Al₂O₃, B₂O₃/SiO₂/Al₂O₃ oder Fe₂O₃/Al₂O₃. Der Rheniumanteil beträgt bei diesen Katalysatoren von 1 bis 20 Massen-%, vorzugsweise von 5 bis 15 Massen-%. Zusätzlich können diese Katalysatoren zur Einstellung der Acidität Alkali- oder Erdalkaliverbindungen aufweisen. Beispiele zur Herstellung und Anwendung solcher Katalysatoren gibt es z. B. in DE 014 42 597, EP 0 268 525, EP 0 282 313, EP 0 438 134, EP 0 444 264, EP 0 710 638, EP 0 710 639 (Basis Re₂O₇) oder DE 017 67 082, EP 0 319 065 und EP 0 245 653 (Basis MoO₃), auf deren Inhalt ausdrücklich verwiesen wird.

Der Verfahrensschritt d) wird vorzugsweise bei einer Temperatur von 20 bis 200 °C, im Falle der Verwendung eines Katalysators auf Basis von Re₂O₇ bevorzugt bei einer Temperatur von 20 bis 120 °C, besonders bevorzugt bei einer Temperatur von 50 bis 100 °C, durchgeführt. Die Metathese der 1-Olefine kann in der flüssigen Phase, in der Gasphase oder in der Flüssig-Gas-Mischphase durchgeführt werden. Bei der Umsetzung in Strömungsrohren betragen die Reaktionszeiten vorzugsweise von 5 bis 40 Minuten, besonders bevorzugt von 10 bis 25 Minuten. Weiterhin ist es möglich, die Metathese gemäß Verfahrensschritt d) in einer Reaktivdestillationskolonne durchzuführen.

Der Umsatz beträgt im Verfahrensschritt d) vorzugsweise von 25 bis 80 %, besonders bevorzugt von 35 bis 60 %. Das aus der Verfahrensstufe d) erhaltene Reaktionsgemisch wird bevorzugt destillativ aufgearbeitet. Nicht umgesetzte(s) 1-Olefin(e) kann/können in die Verfahrensstufe d) zurückgeführt werden. Es kann vorteilhaft sein, wenn ein Teilstrom des in Verfahrensschritt d) erhaltenen Reaktionsgemisches direkt in den Reaktor bzw. die Verfahrensstufe rückgeführt wird und der andere Teilstrom z. B. destillativ aufgearbeitet wird Das entstandene Ethylen kann für bekannte Synthesen genutzt werden oder optional bei allein stehenden Anlagen zu C₄-Olefinen umgesetzt werden, die wiederum als Ausgangsstoff genutzt werden können.

Eine spezielle bevorzugte Ausführungsform der vorliegenden Erfindung ist ein Verfahren zur Herstellung eines Gemisches von isomeren Octenen aus einem C₄-Kohlenwasserstoffgemisch, das als Olefine Isobuten und lineare Butene enthält. Dabei werden die Olefine im Einsatz-Kohlenwasserstoffgemisch durch Hydroformylierung zu den entsprechenden C₅-Aldehyden umgesetzt, die nach Hydrierung und Wasserabspaltung ein Gemisch isomerer 1-Pentene ergeben. Deren Metathese ergibt unter Ethylenabspaltung die Zielolefine, insbesondere 4-Octen bzw. Octen-Gemische.

Durch das erfindungsgemäße Verfahren lässt sich nun auch Isobuten zur Herstellung von Weichmacheralkoholen verwenden. Bei der herkömmlichen Herstellung von Weichmacheralkoholen bzw. deren Ausgangsprodukten, den C₈-Olefinen, wird das Isobuten üblicherweise zunächst aus dem C₄-Olefin-haltigen Strom abgetrennt, da eine Dimerisierung in Anwesenheit von Isobuten zu hochverzweigten C₈-Olefinen führt. Insbesondere die Dimerisierung von 2 Isobuten-Molekülen und anschließende Oxierung und Hydrierung führt zu 3,5,5-Trimethylhexanol, welches einen Isoindex von 3 aufweist. Daraus hergestellte Weichmacher weisen eine sehr hohe Viskosität auf, weshalb sie heute kaum noch eingesetzt werden. Im erfindungsgemäßen Verfahren kann das Isobuten leicht und praktisch nebenproduktfrei durch Hydroformylierung zu 3-Methylbutanal umgesetzt werden. Hydrierung und endständige Dehydratisierung liefert 3-Methylbuten-1. Aus zwei Molekülen 3-Methylbuten-1 ergibt sich in der Metathese 2,5-Dimethylhexen-3 und Ethen. Die Hydroformylierung von 2,5-Dimethylhexen-3 und anschließende Hydrierung liefert als Hauptprodukt 3,6-Dimethylheptanol. Das innenständige Produkt, 2-Isopropyl-4-methyl-pentanol wird üblicherweise nur in geringen Mengen gebildet Deutlich zu erkennen ist der Vorteil des erfindungsgemäßen Verfahrens, denn es wird ein Alkoholgemisch mit einem Isoindex nahe 2 erhalten. Dieses liefert, umgesetzt zu Weichmachern, wie z. B. Di-C₉-Phthalat, einen Weichmacher, der bezüglich der anwendungstechnischen Eigenschaften, wie z. B. der Viskosität in der Bandbreite von üblichen Marktprodukten liegt.

Der Isoindex bzw. die Viskosität kann noch weiter verbessert werden, wenn das bei der Metathese entstehende Ethen zu 1-Buten dimerisiert und als Rohstoff in den Verfahrensschritt a) zurückführt wird. Nach dem ausführlich diskutierten Schema entstehen dann auch lineare Octene (Isoindex 0) und ein Teil des Isobutens wird in Methylheptene (Isoindex 1) eingebaut.

Der Vollständigkeit halber sei noch auf eine weitere Einsparmöglichkeit von Rohstoffen hingewiesen. Rohes Crack-C₄ enthält natürlich je nach Herkunft wechselnde Mengen an gesättigten Butanen, n-Butan und Isobutan. Diese bleiben nach vollständiger Reaktionssequenz als Inerte übrig, man erhält das Rohbutan, das im Gegensatz zur konventionellen Fahrweise kaum mehr reaktive Komponenten enthält. Schließlich werden wie bei jedem technischen Verfahren auch gewisse Mengen an nicht verwertbaren Nebenprodukten gebildet Dies wurde bisher nicht diskutiert, ist aber jedem Fachmann geläufig. Beispielsweise kann die Hydroformylierung zwar hochselektiv durchgeführt werden, es werden aber immer kleine Mengen an Hochsiedern anfallen, zum Beispiel durch Aldolisierung der in der Hydroformylierung gebildeten Aldehyde. Auch bei der Hydrierung der Aldehyde fallen kleine Mengen an Hochsiedern an sowie in der nachfolgenden Dehydratisierung. Die Dehydratisierung erfolgt zwar weitgehend endständig, zum Beispiel mit bis zu 95 % Selektivität. Der Rest sind aber innenständige Olefine. Dies ergeben dann entsprechend in der Metathese Produkte mit anderer C-Zahl als 2 und 8, und zwar Propen, Butene, Pentene und Hexene. Sind größere Mengen an 2-Methylbuten-1 enthalten, kann die Menge an Nebenprodukten noch weiter anwachsen, da 2-Methylbuten-1 im Gegensatz zu 3-Methylbuten-1 und Penten-1 leicht zum innenständigen Olefin isomerisiert, was in der Metathese zu Nebenprodukten führt.

Da ja der erste Schritt der eigentlichen Reaktionssequenz die Hydroformylierung ist, wird Synthesegas benötigt, ein Gemisch aus Kohlenmonoxid und Wasserstoff im ungefähren Molverhältnis von 1:1. Dieses wird üblicherweise durch partielle Oxidation von Kohlenwasserstoffen hergestellt, zum Beispiel durch partielle Oxidation von schwerem Heizöl. Dieses lässt sich nun ersetzen durch die gesamten Nebenprodukte der Verfahrenskette, da ja nur Nebenprodukte entstehen, die C,H und gegebenenfalls noch O enthalten, wie die Hochsieder aus der Hydroformylierung. Auch die gesamten inerten Kohlenwasserstoffe, Butan, Isobutan und eventuell Reste nicht umgesetzter Olefine, eben das Rohbutan, das am Ende der Kette übrig bleibt, kann zur Erzeugung von Synthesegas eingesetzt werden. Da aus dem Synthesegas in der Metathesestufe letztendlich Ethylen entsteht, werden alle Nebenprodukte letztendlich vollständig in Wertprodukt überführt. Auch die inerten Butane und Isobutan, erhebliche Mengen bei einer Großproduktion, gelangen über Synthesegas auf diese Art in die Wertschöpfungskette ohne aufwendige Verfahren wie Dehydrierung der Butane zu Butenen. Benutzt man schließlich noch das Ethylen zur Herstellung von 1-Buten durch Dimerisierung, hat man eine vollständige Verwertung des gesamten Crack-C₄ zu einem einzigen Produkt ohne Nebenprodukte oder Abfälle, somit eine vollständige Nutzung des Rohstoffs.

Mit dem erfindungsgemäßen Verfahren können Gemische hergestellt werden, die zumindest ein Olefin mit 8 bis 12 Kohlenstoffatomen aufweisen. Mit dem erfindungsgemäßen Verfahren kann z. B. Isoocten hergestellt werden, wobei als Edukt ein C₄-Schnitt eingesetzt wird, der einen Gehalt an Isobuten von größer 3 Gew.%, vorzugsweise größer 10 % Gew.% aufweist. Die entstandenen Zielolefine bzw. das Gemisch können/kann als Vorstufe für die Herstellung von vielen weiteren Stoffen, insbesondere von Aldehyden, Alkoholen und/oder Carbonsäuren, insbesondere zur Herstellung von Weichmacheralkoholen verwendet werden. Beispielsweise entsteht durch Hydroformylierung und anschließende Hydrierung aus einem erfindungsgemäß hergestellten C₈-Olefingemisch (Isoocten) ein Nonanolgemisch (Isononanol), das für die Herstellung von Weichmachern geeignet ist.

Die folgenden Beispiele sollen das erfindungsgemäße Verfahren erläutern, ohne die Anwendungsbreite einzuschränken, die sich aus der Beschreibung und den Patentansprüchen ergibt.

### Beispiel 1: Hydroformylierung von Raffinat I

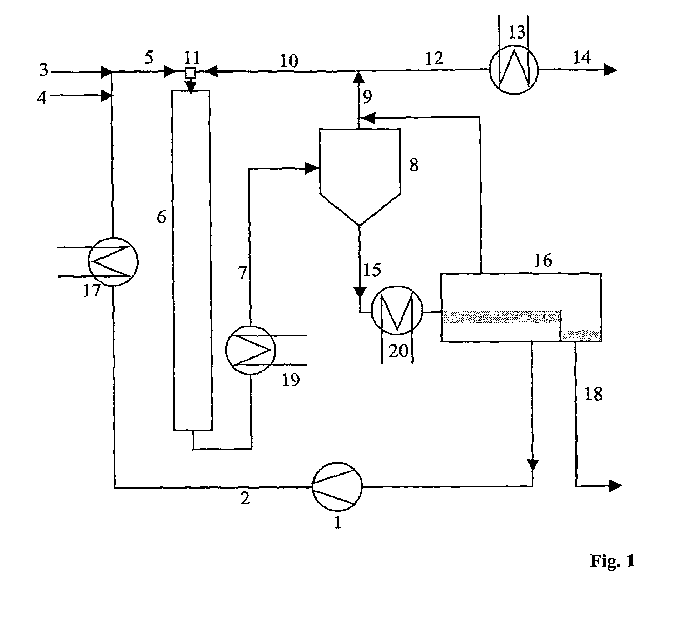

Die Hydroformylierung erfolgte in einer Versuchsapparatur die schematisch in Fig. 1 dargestellt ist. Das Reaktionsrohr **6** hatte eine Länge von 3 m und einen Durchmesser von 17,3 mm (Volumen 705 ml) und enthielt statische Mischelemente der Fa. Sulzer mit einem hydraulischen Durchmesser von 2 mm. Die wässrige Katalysatorlösung **2** wurde mit einer Pumpe **1** im Kreislauf gepumpt. Zur Katalysatorlösung wurde Raffinat **I 3** und Synthesegas **4** zugemischt. Die so erhaltene Mehrphasenmischung **5** wurde über die Mischdüse **11** durch den Rohrreaktor **6** gepumpt. Die resultierende Mischung **7,** bestehend aus Produkt, nicht umgesetztem Edukt und dem Katalysator wurde im Behälter **8** entgast. Das über Leitung **9** entweichende Gasgemisch wurde nicht (Leitung **10** geschlossen, optional aber möglich) in den Reaktor zurückgeführt. Die im Gasstrom 12 enthaltenen C₄-Kohlenwasserstoffe wurden im Kühler **13** im Temperaturbereich von -50 bis -78 °C kondensiert **14.** Der nach der Entgasung im Behälter **8** anfallende Flüssigkeitsstrom **15** wurde in einen Phasentrennbehälter **16** geleitet. Hier wurde die wässrige Katalysatorphase **2** abgetrennt und erneut dem Kreislauf zugeführt. Die Reaktionswärme konnte elegant über außenliegende Wärmetauscher **(17, 19** und **20)** abgeführt werden. Die organische Phase **18** enthielt das hydroformylierte Produkt

Die eingesetzte Katalysatorlösung bestand aus folgenden Komponenten:
290,3 g TPPTS (Triphenylphosphintrisulfonat) in Form des Trinatriumsalzes (0,511 Mol)
403 g Ethylenglycol
318 g Wasser
1,88 g Rhodiumacetat (0,0085 Mol)
Damit betrug die Rhodiumkonzentration in der Katalysatorphase 800 Massen-ppm und das molare Rhodium/Phosphorverhältnis lag bei 1/60.

Als Synthesegas wurde ein Gemisch aus 50 Vol.-% Wasserstoff und 50 Vol.-% Kohlenstoffmonoxid verwendet.

Das eingesetzte Raffinat I hatte folgende Zusammensetzung:

| | |
|---|---|
| Isobutan | 3,10 Massen-% |
| n-Butan | 7,90 Massen-% |
| trans-2-Buten | 8,80 Massen-% |
| 1-Buten | 28,10 Massen-% |
| Isobuten | 45,54 Massen-% |
| Cis-2-Buten | 6,48 Massen-% |
| Sonstiges | 0,08 Massen-% |

Durch den Rohrreaktor wurden bei 115 °C und bei einem Druck von 50 bar je Stunde 400 kg Katalysatorlösung, 600 NI/h Synthesegas und 3 kg Raffinat I geleitet. Nach Abtrennung der Katalysatorphase erhielt man einschließlich des Gaskondensats 3340 g Produktphase mit folgender Zusammensetzung (in Massen-%).

| | |
|---|---|
| i-Butan | 2,78 |
| n-Butan | 7,10 |
| trans-Buten | 7,90 |
| 1-Buten | 8,41 |
| iso-Buten | 38,86 |
| cis-Buten | 5,82 |
| 2-Methylbutanal | 1,17 |
| 3-Methylbutanal | 3,04 |
| n-Pentanal | 24,10 |
| 2-Methylbutanol | 0,06 |
| Methylpropyl-Dioxolan | 0,07 |
| 1-Pentanol | 0,27 |
| 2-n-Butyl 1,3Dioxolan | 0,31 |
| Sonstiges | ca. 0,1 |
| Ethylenglykol | <0,01 |

Dies entsprach im geraden Durchgang einem 1-Buten-Umsatz von 66,66 %. Die Selektivität zu endständig hydroformylierten Produkten (n-Pentanal, n-Pentanol, 2-n-Butyl-1,3-Dioxolan war 95, 4 %. Isobuten wurde zu 5 % umgesetzt, wobei die Selektivität für die Bildung des durch endständige Hydroformylierung entstandenen 3-Methylbutanals größer als 99 % war. Eine Umsetzung der 2-Butene konnte nicht festgestellt werden.

Wegen Fehlen einer geeigneten Druckdestillationsapparatur wurde die destillative Auftrennung des Reaktionsgemisches nicht durchgeführt. Bei der technischen Produktion würde das Synthesegas gegebenenfalls zusammen mit einen Teil der C₄-Kohlenwasserstoffe abgetrennt und in den Rohrreaktor zurückgeführt werden. Das zurückbleibende Gemisch würde in ein Rohaldehydgemisch, in eine Isobutan-Fraktion (Sp = -11,4 °C), eine Fraktion mit Isobuten (Sp = - 6,9 °C) und 1-Buten (Sp = -6,3 °C) sowie eine Fraktion mit cis-2-Buten ( Sp = 3,7 °C), trans-2-Buten (Sp = 0,9 °C9 und mit n-Butan (Sp = -0,5 °C) getrennt.

Die Fraktion mit den nicht umgesetzten 1-Olefinen (1-n-Buten, Isobuten) könnte in den Rohrreaktor zurückgeführt werden, sodass ein vollständiger Umsatz der 1-Olefine im Rohrreaktor möglich ist. Die Fraktion mit den 2-Butenen könnte in einer zweiten Hydroformylierungsstufe (siehe Beispiel 2) eingesetzt werden. Vom Rohaldehyd könnten destillativ die Dioxolane abgetrennt und in den Rohrreaktor eingebracht werden. Diese stehen im Gegenwart der wässrigen Katalysatorlösung mit den zugrundeliegenden Aldehyden im Gleichgewicht. Die destillative Trennung solcher Gemische ist Stand der Technik.

Dieses Beispiel zeigt, dass die beiden α-Olefine (Isobuten und 1-Buten) ohne Umsatz der im Gemisch vorliegenden 2-Butene in Selektivitäten von über 95 % zu durch endständige Hydroformylierung entstandenen Produkten umgesetzt werden und dass bei Rückführung der nicht umgesetzten α-Olefine ein vollständiger Umsatz möglich ist

### Beispiel 2: Hydroformylierung von 2-Butenen

Um aus 2-Butenen ein Hydrofoanylierungsgemisch mit hohem Anteil an n-Pentanal herzustellen, also einem Produkt, das durch endständige Hydroformylierung entstanden ist, muss ein Katalysator eingesetzt werden, der die Verschiebung der Doppelbindungen in den linearen Butenen unter Hydroformylierungsbedingungen bewirkt; denn n-Pentanal kann nur aus 1-Buten, das mit den 2-Butenen im Gleichgewicht steht, gebildet werden.

Bei Verwendung eines schnell isomerisierenden Hydroformylierungskatalysators ist es gleich, ob 1-Buten, trans-2-Buten, cis-2-Buten oder ein beliebiges Gemisch der linearen Butene als Ausgangsstoff gewählt wird, es stellt sich immer ein Gleichgewicht zwischen den Doppelbindungsisomeren ein.

Daher wurde in Beispiel 2 auch kein 2-Buten/n-Butan-Gemisch, wie er aus dem Reaktionsaustrag des Beispiels 1 hätte abgetrennt werden können, eingesetzt, sondern es wurde ein technisch verfügbarer Strom (bezeichnet als Rohbutan) mit folgender Zusammensetzung verwendet.

| | |
|---|---|
| n-Butan | 62 Massen-% |
| trans-2-Buten | 24 Massen-% |
| cis-2-Buten | 11 Massen-% |
| 1-Buten | 3 Massen-% |

Die Hydroformylierungsversuche wurden in einem mit Druckkonstanthaltung, Durchflussmessung , Flügelrührer und HPLC-Pumpe ausgestatteten 100 ml Autoklaven der Fa. Parr durchgeführt. In den Autoklaven wurde unter Argonatmosphäre eine Lösung von Rh-Nonanoat (Firma Chempur) in Toluol mit einem Gehalt von 0.0231 g Rh-Nonanoat und eine Lösung von 0,2285 g des Liganden A (hergestellt in Anlehnung an US 4,885,401, UCC) in Toluol eingefüllt Die Toluolmenge wurde dann auf 42 g ergänzt

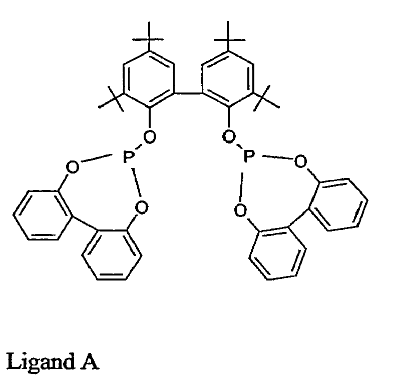

Anschließend wurde das Rhodium-Ligand-Gemisch unter Rühren (1000 U/min) mit Synthesegas (CO: H₂ 1:1) auf einen Druck von 5 - 10 bar (Solldruck 25 bar) gebracht und auf 120 °C aufgeheizt. Nach Erreichen der gewünschten Reaktionstemperatur wurden 30ml (18,8 g) Rohbutan mit der oben angegebenen Zusammensetzung über eine HPLC-Pumpe zudosiert und der Druck auf den Solldruck von 25 bar nachgeregelt. Während der Versuchslaufzeit wurden in festgelegten Zeitabständen Proben gezogen. Die Reaktion wurde bei konstantem Druck (Druckregler der Fa. Bronkhorst (NL)) über 5,5 Stunden geführt. Der Autoklav wurde nach Ablauf der Versuchszeit auf Raumtemperatur abgekühlt, entspannt und mit Argon gespült Jeweils 0,2 ml der Autoklavenlösung wurden mit 0,8 ml n-Pentan versetzt und gaschromatographisch analysiert

| Beispiel- Nr. | Rh-Konzentration | Molares Ligand- | Zeit [h] | Temperatur [°C] | Druck [bar] | Umsatz [%] | Selelektivität [%] |
|---|---|---|---|---|---|---|---|
| | [ppm] | Rh-Verhältnis | | | | | a) zu Pentanalen |
| | | | | | | | b) zu n-Pentanal |
| 2a | 100 | 5/1 | 3,5 | 120 | 25 | 85,8 | a)100 |
| | | | | | | | b) 82 |
| 2b | 100 | 5/1 | 5,5 | 120 | 25 | 95,3 | a) 100 |
| | | | | | | | b) 82 |

Beispiel 2 zeigt, dass aus dem Edukt, das nur einen geringen Anteil an 1-Buten aufweist, mit hoher n-Selektivität und hohen Umsätzen n-Valeraldehyd erzeugen werden kann.

Die beiden Beispiele 1 und 2 belegen, dass ein Gemisch von C₄-Olefinen weitgehend zu Produkten umgesetzt werden können, die durch endständige Hydroformylierung und gegebenenfalls einer Folgereaktion entstanden sind.

Die Hydrierung von durch Hydroformylierung gewonnenen Aldehyden oder von Hydroformylierungsgemischen ist allgemeiner Stand der Technik. Die in den Beispielen 1 und 2 angefallenen Hydroformylierungsgemische wurden dennoch nicht hydriert und zu den entsprechenden Pentanolen aufgearbeitet sondern es wurden der Einfachheit halber in den Dehydratisierungsversuchen (Beispiel 4-5) technisch verfügbare Pentanole ( Fa. Celanese) eingesetzt.

### Beispiel 3: Herstellung eines Dehydratisierungskatalysators

Als Trägermaterial für die Herstellung eines Dehydratisierungskatalysators wurde ein saures γ-Aluminiumoxid mit einem Na₂O-Gehal von kleiner 300 Massen-ppm der Fa. Axens verwendet. Dieses Aluminiumoxid mit einer BET-Obertläche von 225 m²/g und einem Porenvolumen von 0,68 ml/g lag als Extrudat (Zylinder mit einer Länge von 4-6 mm und einem Durchmesser von 1,25 mm) vor. Als Barium-Vorläufer für die basische Modifizierung des Aluminiumoxids mit Bariumoxid (BaO) wurde Bariumnitrat Ba(NO₃)₂ eingesetzt.

Vor der Aufbringung des Bariumsalzes wurde das Aluminiumoxid zuerst bei 90 °C 5 h lang in einem Umlufttrockenschrank getrocknet. 200 g des getrockneten Strangextrudats wurden anschließend in einer Rotationstrommel (Drageetrommel) bei Raumtemperatur mit einer Lösung, bestehend aus 130 ml Wasser und 5,19 g Bariumnitrat, mit Hilfe einer Sprühdüse imprägniert.

Nach der Imprägnierung wurde das mit dem Bariumsalz beladene Extrudat zuerst bei 110 °C 5 h lang in einem Umlufttrockenschrank getrocknet Die anschließende Calcinierung, bei der das Bariumsalz zu Bariumoxid bzw. zu einer Barium/Aluminium/Sauerstoffverbindung umgewandelt wird, erfolgte in einem Wirbelbettreaktor im Luftstrom für 10 h bei 450 °C. Der fertige Katalysator enthielt 1,5 Massen-% Bariumverbindungen, berechnet als Bariumoxid.

### Beispiel 4: Herstellung von 1-Penten aus 1-Pentanol

1-Pentanol wurde in einem elektrisch beheizten Durchfluss-Festbettreak-tor am nach Beispiel 3 hergestellten Katalysator umgesetzt. Vor dem Eintritt in den Reaktor wurde das flüssige Edukt in einem vorgeschalteten Verdampfer bei 220 °C verdampft. Bei einer Reaktionstemperatur von 350 °C wurden stündlich 24 g 1-Pentanol durch 15,1 g Katalysator in der Gasphase, entsprechend einem WHSV-Wert von 1,59 h⁻¹, durchgeleitet. Das gasförmige Produkt wurde in einem Kühler abgekühlt und in einer Glasvorlage gesammelt. Das Produkt hatte wasserfrei gerechnet folgende, gaschromatographisch bestimmte Zusammensetzung:

| | |
|---|---|
| 1-Penten | 93,1 Massen-% |
| trans 2-Penten | 1,9 Massen-% |
| cis-2-Penten | 4,1 Massen-% |
| 1-Pentanol | 0,1 Massen-% |
| sonstige Stoffe | 0,8 Massen-% |

### Beispiel 5: Herstellung von 3-Methylbut-1-en aus 3-Methylbutan-1-ol

3-Methylbutan-1-ol wurde in einem elektrisch beheizten Durchfluss-Festbettreaktor am nach Beispiel 3 hergestellten Katalysator umgesetzt. Vor dem Eintritt in den Reaktor wurde das flüssige Edukt in einem vorgeschalteten Verdampfer bei 220 °C verdampft Bei einer Reaktionstemperatur von 340 °C wurden stündlich 24 g 3-Methylbutan-1-ol durch 15,1 g Katalysator in der Gasphase, entsprechend einem WHSV-Wert von 1,59 h⁻¹, durchgeleitet. Das gasförmige Produkt wurde in einem Kühler abgekühlt und in einer Glasvorlage gesammelt. Das Produkt hatte wasserfrei gerechnet folgende Zusammensetzung:

| | |
|---|---|
| 3-Methylbut-1-en | 91,1 Massen-% |
| cis-2-Methylbut-2-en | 4,5 Massen-% |
| trans-2-Methylbut-2-en | 2,5 Massen-% |
| 3-Methylbutan-1-ol | 1,3 Massen-% |
| sonstige Stoffe | 0,6 Massen-% |

### Beispiel 6: Herstellung eines Rhenium/Alummiurnoxid-Metathesekatalysator

Ein Gemisch aus 20 g γ-Aluminiumoxid-Tabletten, 1,39 g Rheniumheptoxid ( Re₂O₇) (Firma Merck),270 ml 1,4-Dioxan und 30 ml Wasser wurde 16 Stunden lang unter Rückfluss gekocht Der Ansatz wurde mit Hilfe eines Rotationsverdampfer bei Temperaturen bis 80 °C und einem Vakuum bis 20 mbar zur Trockne eingedampft. Der zurückbleibende Feststoff wurde in einen Metathesereaktor gefüllt und darin bei 120 °C im Stickstoffstrom (5 1/h) eine Stunde lang vorgetrocknet. Anschließend wurde im Stickstoffstrom die Temperatur auf 550 °C erhöht. Nach Erreichen dieser Temperatur wurde bei gleichbleibenden Gasdurchsatz auf ein Gasgemisch, bestehend aus 20 % Sauerstoff und 80 % Stickstoff, umgestellt. Nach 16 Stunden bei 550 °C wurde der Katalysator im Stickstoffstrom (5 1/h) auf Reaktionstemperatur abgekühlt. Der Katalysator enthielt 6,5 Massen-% Rheniumoxid.

### Metatheseversuche (Beispiele 7 bis 9)

Der Einsatzstoff (Pentene) wurde aus einer auf -15 °C gekühlten Vorlage, die zur Quantifizierung der eingesetzten Masse (bzw. des Massenflusses) auf einer Waage steht, mit Hilfe einer Schlauchpumpe über einen Verdampfer (Rundkolben in einem 100 °C heißen Ölbad) auf den Kopf eines Metathesereaktors gefördert. Dieser Reaktor bestand aus einem Rohr mit der Länge von 500 mm und einem Durchmesser von 24 mm. Der Reaktor wurde mit Hilfe einer Mantelheizung thermostatisiert. Aus dem den Reaktor verlassenden Gemisch wurden die flüssigen Produkte bei -15 °C auskondensiert, ausgewogen und gaschromatographisch analysiert.

### Beispiel 7: Metathese von 1-Penten

Über 21,4g Rheniumoxid/Aluminiumoxidkatalysator (Beispiel 6) wurde bei einer Temperatur von 100 °C 1-Penten mit einer Geschwindigkeit von 95 g/h geleitet. Der 1-Penten-Umsatz betrug dabei 35 % und die Selektivität zu 4-Octenen 98,7 %.

### Beispiel 8: Metathese von 3-Methylbut-1-en

Über 21,4 g Rheniumoxid/Aluminiumoxidkatalysator (Beispiel 6) wurde bei einer Temperatur von 100 °C 3-Methylbut-1-en mit einer Geschwindigkeit von 95 g/h geleitet. Der 3-Methylbut-1-en-Umsatz betrug dabei 35 % und die Selektivität zu 2,5-Dimethyl-3-hexenen über 99 %.

### Beispiel 9: Metathese eines Gemisches aus 1-Penten und 3-Methylbut-l-en

Über 21,4 g Rheniumoxid/Aluminiumoxidkatalysator (Beispiel 6) wurde bei einer Temperatur von 100 °C ein äqimolares Gemisch aus 1-Penten und 3-Methylbut-1 en mit einer Geschwindigkeit von 95 g/h geleitet. Der Umsatz der beiden Einsatzolefine lag bei 35 % und die Ausbeute an C₈-Olefinen bei 99 %. Die C₈-Olefinfraktion bestand zu 25 % aus 4-Octenen, zu 25 % aus 2,5-Dimethyl-3-hexenen und zu 50 % aus 2-Methylhept-3-enen.

## Patentansprüche

1. Verfahren zur Herstellung zumindest eines Olefins mit 8 bis 12 Kohlenstoffatomen aus zumindest einem Olefin mit 4 bis 6 Kohlenstoffatomen durch eine vierstufige Synthese,
**dadurch gekennzeichnet, dass**
a) in einem im ersten Verfahrensschritt zumindest ein Einsatzolefin hydroformyliert wird,
b) der im ersten Schritt a) erhaltenen zumindest eine Aldehyd zu dem entsprechenden Alkohol hydriert wird,
c) aus dem im zweiten Verfahrensschritt b) erhaltenen zumindest einem Alkohol durch Wasserabspaltung zumindest ein 1-Olefin hergestellt wird und
d) aus dem im dritten Verfahrensschritt c) erhaltenen zumindest einem 1-Olefin(en) durch Metathese unter Ethylenabspaltung zumindest ein Olefin gewonnen wird.

2. Verfahren nach Anspruch 1,
**dadurch gekennzeichnet,**
**dass** ein Gemisch von Olefinen mit 4 bis 6 Kohlenstoffatomen eingesetzt wird und ein Gemisch von Olefinen mit 8 bis 12 Kohlenstoffatomen erhalten wird.

3. Verfahren nach Anspruch 1 oder 2,
**dadurch gekennzeichnet,**
**dass** im zweiten Verfahrensschritt b) als Katalysator ein Nickel-, Kupfer-, Kupfer/Nickel-, Kupfer/Chrom-, Kupfer/Chrom/Nickel-, Zink/Chrom-, Nickel/Molybdän-Katalysator eingesetzt wird.

4. Verfahren nach zumindest einem der Ansprüche 1 bis 3,
**dadurch gekennzeichnet,**
**dass** im dritten Verfahrensschritt c) die Wasserabspaltung kontinuierlich an einem festen Katalysator durchgeführt wird, der formal aus Aluminiumoxid und Bariumoxid besteht.

5. Verfahren nach zumindest einem der Ansprüche 1 bis 4,
**dadurch gekennzeichnet,**
**dass** im vierten Verfahrensschritt d) ein Rheniumkatalysator, der Re₂O₇ auf γ-Al₂O₃ oder auf Mischträgern, ausgewählt aus SiO₂/Al₂O₃, B₂O₃/SiO₂/Al₂O₃ oder Fe₂O₃/Al₂O₃

6. Verfahren nach zumindest einem der Ansprüche 1 bis 5,
**dadurch gekennzeichnet,**
**dass** als Edukt in Verfahrensschritt a) ein Kohlenwasserstoffstrom eingesetzt wird, der Isobuten und lineare Butene aufweist oder aus diesen besteht.

7. Verfahren nach zumindest einem der Ansprüche 1 bis 6,
**dadurch gekennzeichnet,**
**dass** als Edukt ein C₄-Schnitt, ausgewählt aus Raffinat I, selektiv hydriertem Crack-C₄, C₄-Schnitten aus FCC-Anlagen oder C₄-Olefinen, hergestellt durch Fischer-Tropsch-Synthese, eingesetzt wird.

8. Verfahren nach zumindest einem der Ansprüche 1 bis 7,
**dadurch gekennzeichnet,**
**dass** als Edukt technische C₄-Schnitte eingesetzt werden, die einen Isobuten-Gehalt von größer 3 Gew.-% aufweisen.

9. Verfahren nach zumindest einem der Ansprüche 6 bis 8,
**dadurch gekennzeichnet,**
**dass** aus der nach dem dritten Verfahrensschritt c) erhaltenen 1-Olefinfraktion, die Olefine mit 5 Kohlenstoffatomen enthält, 3-Methylbut-1-en abgetrennt wird.

## Claims

1. Process for preparing at least one olefin having from 8 to 12 carbon atoms from at least one olefin having from 4 to 6 carbon atoms by means of a four-stage synthesis, **characterized in that**
a) at least one starting olefin is hydroformylated in the first process step,
b) the at least one aldehyde obtained in the first step a) is hydrogenated to form the corresponding alcohol,
c) at least one 1-olefin from the at least one alcohol obtained by elimination of water is prepared in the second process step b) and
d) at least one olefin is obtained by metathesis with elimination of ethylene from the at least one 1-olefin(s) obtained in the third process step c).

2. Process according to Claim 1,
**characterized in that** a mixture of olefins having from 4 to 6 carbon atoms is used and a mixture of olefins having from 8 to 12 carbon atoms is obtained.

3. Process according to Claim 1 or 2,
**characterized in that** a nickel, copper, copper/nickel, copper/chromium, copper/chromium/nickel, zinc/chromium, nickel/molybdenum catalyst is used as catalyst in the second process step b).

4. Process according to at least one of Claims 1 to 3,
**characterized in that** the elimination of water in the third process step c) is carried out continuously over a solid catalyst which consists formally of aluminum oxide and barium oxide.

5. Process according to at least one of Claims 1 to 4,
**characterized in that** a rhenium catalyst comprising Re₂O₇ on γ-Al₂O₃ or on mixed supports selected from among SiO₂/Al₂O₃, B₂O₃/SiO₂/Al₂O₃ or Fe₂O₃/Al₂O₃ is used in the fourth process step d).

6. Process according to at least one of Claims 1 to 5,
**characterized in that** a hydrocarbon stream comprising or consisting of isobutene and linear butenes is used as starting material in process step a).

7. Process according to at least one of Claims 1 to 6,
**characterized in that** a C₄ fraction selected from among raffinate I, selectively hydrogenated C₄ fraction from a cracker, C₄ fractions from FCC plants or C₄-olefins prepared by the Fischer-Tropsch synthesis is used as starting material.

8. Process according to at least one of Claims 1 to 7,
**characterized in that** industrial C₄ fractions having an isobutene content of greater than 3% by weight are used as starting material.

9. Process according to at least one of Claims 6 to 8,
**characterized in that** 3-methyl-1-butene is separated off from the 1-olefin fraction comprising olefins having 5 carbon atoms which is obtained after the third process step c).

## Revendications

1. Procédé en vue de la fabrication d'au moins une oléfine, ayant de 8 à 12 atomes de carbone à partir d'au moins une oléfine ayant de 4 à 6 atomes de carbone, grâce à une synthèse à quatre étapes, **caractérisé**
a) **en ce que** l'on procède, en une fois, dans la première étape de procédé, à l'hydroformylation d'au moins une oléfine de charge,
b) **en ce que** l'on procède à l'hydrogénation du au moins un aldéhyde obtenu dans la première étape a) pour former l'alcool correspondant,
c) **en ce que** l'on prépare, par clivage d'eau, au moins une 1-oléfine à partir du au moins un alcool obtenu dans la deuxième étape de procédé b) et
d) **en ce que** l'on obtient, par métathèse sous clivage d'éthylène, au moins une oléfine, à partir de la au moins une 1-oléfine obtenue dans la troisième étape de procédé c).

2. Procédé selon la revendication 1, **caractérisé en ce que** l'on utilise un mélange d'oléfines ayant de 4 à 6 atomes de carbone et que l'on obtient un mélange d'oléfines ayant de 8 à 12 atomes de carbone.

3. Procédé selon la revendication 1 ou 2, **caractérisé en ce que**, dans la deuxième étape de procédé b), l'on utilise en tant que catalyseur un catalyseur au nickel, au cuivre, au cuivre/nickel, au cuivre/chrome, au cuivre/chrome/nickel, au zinc/chrome, au nickel/molybdène.

4. Procédé selon au moins l'une des revendications 1 à 3, **caractérisé en ce que**, dans la troisième étape de procédé c), l'on effectue le clivage d'eau en continu sur un catalyseur solide, qui se compose formellement d'oxyde d'aluminium et d'oxyde de baryum.

5. Procédé selon au moins l'une des revendications 1 à 4, **caractérisé en ce que**, dans la quatrième étape de procédé d), l'on utilise un catalyseur au rhénium, qui présente du Re₂O₇ sur du γ-Al₂O₃ ou sur des supports mixtes, sélectionnés parmi SiO₂ /Al₂O₃, B₂O₃/SiO₂/Al₂O₃ ou Fe₂O₃/Al₂O₃.

6. Procédé selon au moins l'une des revendications 1 à 5, **caractérisé en ce que** l'on utilise, en tant que produit de départ dans l'étape de procédé a), un courant d'hydrocarbures, qui présente de l'isobutène et des butènes linéaires ou qui se compose de ceux-ci.

7. Procédé selon au moins l'une des revendications 1 à 6, **caractérisé en ce que** l'on utilise, en tant que produit de départ, une coupe en C₄, sélectionnée parmi le raffinat I, un produit de craquage en C₄ hydrogéné sélectivement, des coupes en C₄ provenant d'installations FCC ou des oléfines en C₄, produits par synthèse Fischer-Tropsch.

8. Procédé selon au moins l'une des revendications 1 à 7, **caractérisé en ce que** l'on utilise, en tant que produit de départ, des coupes techniques en C₄, qui présentent une teneur en isobutène de plus de 3 % en poids.

9. Procédé selon au moins l'une des revendications 6 à 8, **caractérisé en ce que** l'on sépare le 3-méthylbut-1-ène de la fraction de 1-oléfines obtenue selon la troisième étape de procédé c), qui contient des oléfines ayant 5 atomes de carbone.
